(19) Europäisches Patentamt
European Patent Office
Office européen des brevets

(11) EP 4 435 893 A1

(12) EUROPEAN PATENT APPLICATION

(43) Date of publication:
**25.09.2024 Bulletin 2024/39**

(21) Application number: **23209084.5**

(22) Date of filing: **10.11.2023**

(51) International Patent Classification (IPC):
***H01M 4/485*** *(2010.01)* ***H01M 10/0525*** *(2010.01)*
***H01M 10/0567*** *(2010.01)* ***H01M 10/0569*** *(2010.01)*

(52) Cooperative Patent Classification (CPC):
**H01M 10/0567; H01M 4/485; H01M 10/0525; H01M 10/0569**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.03.2023 KR 20230038065**

(71) Applicant: **Samsung SDI Co., Ltd.**
**Yongin-si, Gyeonggi-do 17084 (KR)**

(72) Inventors:
- **Kim, Sundae**
**17084 Yongin-si, Gyeonggi-do (KR)**
- **Yang, Yeji**
**17084 Yongin-si, Gyeonggi-do (KR)**
- **Kim, Sanghoon**
**17084 Yongin-si, Gyeonggi-do (KR)**
- **Choi, Hyunbong**
**17084 Yongin-si, Gyeonggi-do (KR)**
- **Kim, Dahyun**
**17084 Yongin-si, Gyeonggi-do (KR)**
- **Park, Hongryeol**
**17084 Yongin-si, Gyeonggi-do (KR)**
- **Park, Sangwoo**
**17084 Yongin-si, Gyeonggi-do (KR)**

(74) Representative: **Gulde & Partner**
**Patent- und Rechtsanwaltskanzlei mbB**
**Wallstraße 58/59**
**10179 Berlin (DE)**

# (54) RECHARGEABLE LITHIUM BATTERY

(57) Provided is a rechargeable lithium battery including an electrolyte including a non-aqueous organic solvent, a lithium salt, and an additive; positive electrode including a positive electrode active material; and a negative electrode including a negative electrode active material, wherein the non-aqueous organic solvent includes less than about 5 wt% of ethylene carbonate based on the total weight of the non-aqueous organic solvent, the positive electrode active material includes a lithium nickel manganese-based oxide, and the additive comprises a compound represented by Chemical Formula 1.

EP 4 435 893 A1

## Description

## BACKGROUND

### 1. Field

Embodiments of this disclosure relate to a rechargeable lithium battery.

### 2. Description of Related Art

Rechargeable lithium batteries may be recharged and may have three or more times as high energy density per unit weight as a lead storage battery, nickel-cadmium battery, nickel hydrogen battery, nickel zinc battery and/or the like. Rechargeable lithium batteries may be also charged at a high rate and thus, are commercially manufactured for a laptop, a cell phone, an electric tool, an electric bike, and the like, and research on improvement of additional energy density of rechargeable lithium batteries has been conducted.

As information technology (IT) devices increasingly achieve high performance, a high-capacity battery is desirable, but the high capacity may be realized through expansion of a voltage range, thereby increasing energy density but also bringing about deteriorating performance of a positive electrode due to oxidization of an electrolyte in the high voltage range.

A cobalt-free lithium nickel manganese-based oxide is a positive electrode active material that does not include cobalt but does include nickel, manganese, and/or the like as a main component in its composition, and accordingly, a positive electrode including the same may be economical and realize high energy density and thus draws attention as a next generation positive electrode active material.

However, if the positive electrode including the cobalt-free lithium nickel manganese-based oxide is used in a high voltage environment, transition metals may be eluted due to structural collapse of the positive electrode, thereby causing gas generation inside a cell, capacity reduction, and/or the like. This transition metal elution tends to be aggravated in a high temperature environment, wherein the eluted transition metals are precipitated on the surface of a negative electrode and may cause a side reaction and thereby increase battery resistance and deteriorate battery cycle-life and output characteristics.

Accordingly, if the positive electrode including the cobalt-free lithium nickel manganese-based oxide is used, an electrolyte applicable under high-voltage and high-temperature conditions is desirable.

## SUMMARY

The invention is defined by the appended claims. The description that follows is subjected to this limitation. Any disclosure lying outside the scope of said claims is only intended for illustrative as well as comparative purposes.

Some embodiments of the present disclosure provide a rechargeable lithium battery exhibiting improved high-voltage characteristics and high-temperature characteristics by combining a positive electrode including lithium nickel manganese-based oxide together with an electrolyte capable of effectively protecting the positive electrode including a lithium nickel manganese-based oxide to reduce elution of transition metals under high-voltage and high-temperature conditions and thus to suppress or reduce structural collapse of the positive electrode.

The present invention provides a rechargeable lithium battery including an electrolyte including a non-aqueous organic solvent, a lithium salt, and an additive; a positive electrode including a positive electrode active material; and a negative electrode including a negative electrode active material,

wherein the non-aqueous organic solvent includes less than 5 wt% of ethylene carbonate based on the total weight of the non-aqueous organic solvent,
the positive electrode active material includes a lithium nickel manganese-based oxide, and
the additive includes a compound represented by Chemical Formula 1:

Chemical Formula 1

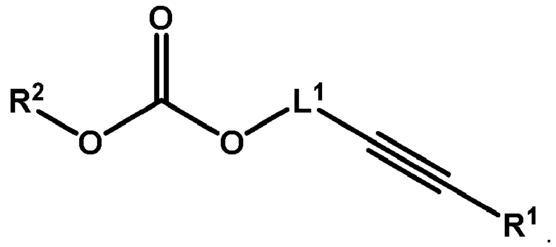

In Chemical Formula 1,

$L^1$ is a substituted or unsubstituted C1 to C3 alkylene group,
$R^1$ is hydrogen or a substituted or unsubstituted C1 to C10 alkyl group, and
$R^2$ is a substituted or unsubstituted C1 to C10 alkyl group, a substituted or unsubstituted C2 to C10 alkenyl group, a substituted or unsubstituted C2 to C10 alkynyl group, or a substituted or unsubstituted C6 to C20 aryl group.

The term "substituted" refers to replacement of at least one hydrogen of a substituent by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group

Some embodiments may realize a rechargeable lithium battery exhibiting improved battery stability and cycle-life characteristics by combining together a positive electrode including a cobalt-free lithium nickel manganese-based oxide with an electrolyte capable of effectively protecting the positive electrode to secure phase transition safety of the positive electrode in a high temperature high voltage environment and to suppress or reduce decomposition of the electrolyte and a side reaction with electrodes and thus reduce gas generation and concurrently, suppress or reduce an increase in battery internal resistance.

## BRIEF DESCRIPTION OF THE DRAWING

The accompanying drawing, together with the specification, illustrates an embodiment of the subject matter of the present disclosure, and, together with the description, serves to explain principles of embodiments of the subject matter of the present disclosure.

The accompanying drawing is a schematic view illustrating a rechargeable lithium battery according to some embodiments.

## DETAILED DESCRIPTION

Hereinafter, a rechargeable lithium battery according to some embodiments of the present disclosure will be described in more detail with reference to the accompanying drawing

In the present specification, if a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

In one example of the present disclosure, "substituted" refers to replacement of at least one hydrogen of a substituent or a compound by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group. In some embodiments, in examples of the present disclosure, "substituted" refers to replacement of at least one hydrogen of a substituent or a compound by deuterium, a halogen, a C1 to C20 alkyl group, a C6 to C30 aryl group, a C1 to C10 fluoroalkyl group, or a cyano group. In some embodiments, in examples of the present disclosure, "substituted" refers to replacement of at least one hydrogen of a substituent or a compound by deuterium, a halogen, a C1 to C5 alkyl group, a C6 to C18 aryl group, a C1 to C5 fluoroalkyl group, or a cyano group. In some embodiments, in examples of the present disclosure, "substituted" refers to replacement of at least one hydrogen of a substituent or a compound by deuterium, a cyano group, a halogen, a methyl group, an ethyl group, a propyl group, a butyl group, a phenyl group, a biphenyl group, a terphenyl group, a trifluoromethyl group, or a naphthyl group.

A rechargeable lithium battery may be classified into a lithium ion battery, a lithium ion polymer battery, or a lithium polymer battery depending on types (or kinds) of a separator and an electrolyte and also may be classified to be cylindrical, prismatic, coin-type, pouch-type, or the like depending on its shape. In some embodiments, the rechargeable lithium battery may be a bulk type or a thin film type depending on its size. Structures and manufacturing methods for batteries pertaining to this disclosure may be any suitable ones generally used in the art.

Herein, a cylindrical rechargeable lithium battery will be described as an example of the rechargeable lithium battery. The accompanying drawing schematically shows the structure of a rechargeable lithium battery according to some embodiments. Referring to the accompanying drawing, a rechargeable lithium battery 100 includes a battery cell including a positive electrode 114, a negative electrode 112 facing the positive electrode 114, a separator 113 between the positive electrode 114 and the negative electrode 112, and an electrolyte impregnating the positive electrode 114, the negative electrode 112, and the separator 113, a battery case 120 housing the battery cell, and a sealing member 140 sealing the battery case 120.

Hereinafter, a more detailed configuration of the rechargeable lithium battery 100 according to some embodiments of the present disclosure will be described.

A rechargeable lithium battery according to the invention includes an electrolyte, a positive electrode, and a negative electrode.

The electrolyte includes a non-aqueous organic solvent, a lithium salt, and an additive, the non-aqueous organic solvent include less than 5 wt% of ethylene carbonate based on the total weight of the non-aqueous organic solvent, and the additive includes a compound represented by Chemical Formula 1.

Chemical Formula 1

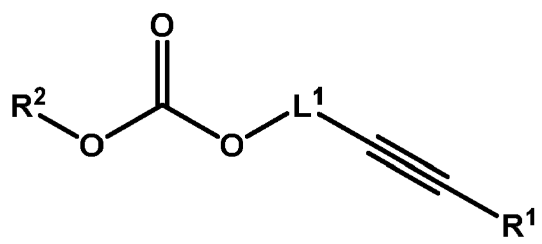

In Chemical Formula 1,

$L^1$ is a substituted or unsubstituted C1 to C3 alkylene group,
$R^1$ is hydrogen or a substituted or unsubstituted C1 to C10 alkyl group, and
$R^2$ is a substituted or unsubstituted C1 to C10 alkyl group, a substituted or unsubstituted C2 to C10 alkenyl group, a substituted or unsubstituted C2 to C10 alkynyl group, or a substituted or unsubstituted C6 to C20 aryl group.

The positive electrode includes a cobalt-free positive electrode active material including a lithium nickel manganese-based oxide.

In a positive electrode active material including a lithium nickel manganese-based oxide, for example, a cobalt-free lithium nickel manganese-based oxide, structural instability is severe under high-voltage conditions, resulting in solvent decomposition and elution of transition metals, for example, Ni.

Due to such a transition metal elution phenomenon, deterioration of performance of a positive electrode and short-circuiting caused by elution of transition metals may occur, resulting in a decrease in cycle-life characteristics of the battery and a rapid increase in resistance.

However, in embodiments of the present disclosure using the aforementioned electrolyte together, it is possible to alleviate or reduce a decrease in the cycle-life characteristics of the battery and the rapid increase in resistance.

By using a positive electrode including a cobalt-free lithium nickel manganese-based oxide in an electrolyte containing less than 5 wt% of ethylene carbonate based on the total weight of the non-aqueous organic solvent, the elution of transition metals can be effectively reduced under high-voltage and high-temperature conditions. Accordingly, the high-voltage characteristics and high-temperature characteristics of the battery can be improved by suppressing or reducing the collapse of the positive electrode structure. For example, the content of ethylene carbonate in the non-aqueous organic solvent may be less than 3 wt%, less than 1 wt% or less then 0.1 wt% based on the total weight of the non-aqueous organic solvent. According to one embodiment, the non-aqueous organic solvent is free of ethylene carbonate (except for indistinguishable traces left by the synthesis of other non-aqueous organic solvents).

The non-aqueous organic solvent serves as a medium for transmitting ions taking part in the electrochemical reaction of a battery.

The non-aqueous organic solvent may be a carbonate-based, ester-based, ether-based, ketone-based, alcohol-based, and/or aprotic solvent.

The carbonate-based solvent may include at least one of dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), and butylene carbonate (BC). The ester-based solvent may include at least one of methyl acetate, ethyl acetate, n-propyl acetate, t-butyl acetate, methylpropionate, ethyl propionate, propylpropionate, decanolide, mevalonolactone, and caprolactone. The ether-based solvent may include at least one of dibutyl ether, tetraglyme, diglyme, dimethoxyethane, 2-methyltetrahydrofuran, and tetrahydrofuran. In some embodiments, the ketone-based solvent may include cyclohexanone. The alcohol-based solvent may include at least one of ethanol, and isopropyl alcohol and the aprotic solvent may include nitriles such as R-CN (wherein R is a hydrocarbon group having a C2 to C20 linear, branched, or cyclic structure and may include a double bond, an aromatic ring, or an ether bond), amides such as dimethyl formamide, dioxolanes such as 1,3-dioxolane, or sulfolanes.

The non-aqueous organic solvent may be used alone or in a mixture, and if the non-aqueous organic solvent is used in a mixture, the mixture ratio may be controlled in accordance with a suitable or desirable battery performance.

The non-aqueous organic solvent includes less than 5 wt% of ethylene carbonate (which is a cyclic carbonate ester) based on the total weight of the non-aqueous organic solvent.

If the content of ethylene carbonate is included in an amount of greater than or equal to 5 wt%, an activity of Ni increases during high-voltage operation, and an oxidation number of Ni tends to be reduced from tetravalent to divalent. Ethylene carbonate, which has low oxidation stability, is oxidatively decomposed, and as a result, Ni is eluted and deposited on the negative electrode.

As an example, the non-aqueous organic solvent may be composed of only chain carbonate. A "chain carbonate" is compound including a carbonate group and two substituted or non-substituted alkyl moieties bond to the carbonate group, but not forming a cycle together with the carbonate group of the compound. In some embodiments, as the resistance increase rate during high-temperature storage is significantly alleviated, excellent high-temperature storage characteristics may be implemented.

As used herein, "composed of only chain carbonate" means including non-aqueous organic solvents belonging to the category of chain carbonates alone or in combination without being mixed together with cyclic carbonates.

In some embodiments, the chain carbonate may be represented by Chemical Formula 2.

Chemical Formula 2

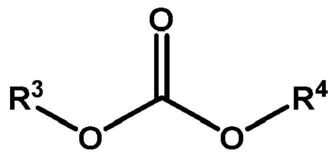

In Chemical Formula 2

$R^3$ and $R^4$ are each independently a substituted or unsubstituted C1 to C20 alkyl group.

For example, $R^3$ and $R^4$ in Chemical Formula 2 may each independently be a substituted or unsubstituted C1 to C10 alkyl group, and for example, $R^3$ and $R^4$ may each independently be a substituted or unsubstituted C1 to C5 alkyl group.

In some embodiments, $R^3$ and $R^4$ in Chemical Formula 2 may each independently be a substituted or unsubstituted methyl group, a substituted or unsubstituted ethyl group, a substituted or unsubstituted n-propyl group, a substituted or unsubstituted n-butyl group, a substituted or unsubstituted n-pentyl group, a substituted or unsubstituted iso-butyl group, or a substituted or unsubstituted neo-pentyl group.

For example, the non-aqueous organic solvent according to some embodiments may be a mixed solvent of at least two solvent selected from dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

The non-aqueous organic solvent according to some embodiments may be dimethyl carbonate (DMC) or a combination of dimethyl carbonate (DMC) and ethylmethyl carbonate (EMC).

The non-aqueous organic solvent may include ethylmethyl carbonate (EMC) and dimethyl carbonate (DMC) in a volume ratio of about 0:100 to about 50:50.

It may be more suitable or desirable in terms of improving battery characteristics that the non-aqueous organic solvent includes dimethyl carbonate (DMC) in an amount of more than about 50 volume%.

For example, the non-aqueous organic solvent may include ethylmethyl carbonate (EMC) and dimethyl carbonate (DMC) in a volume ratio of about 0:100 to about 40:60, about 0:100 to about 30:70, about 10:90 to about 40:60, or about 10:90 to about 30:70.

The non-aqueous organic solvent may further include an aromatic hydrocarbon-based organic solvent in addition to the carbonate-based solvent. Herein, the carbonate-based solvent and the aromatic hydrocarbon-based organic solvent may be mixed together in a volume ratio of about 1:1 to about 30:1.

The aromatic hydrocarbon-based organic solvent may be an aromatic hydrocarbon-based compound represented by Chemical Formula 3.

Chemical Formula 3

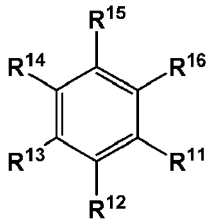

In Chemical Formula 3, $R^{11}$ to $R^{16}$ are the same or different and are selected from hydrogen, a halogen, a C1 to C10 alkyl group, a C1 to C10 haloalkyl group, and a combination thereof.

Examples of the aromatic hydrocarbon-based organic solvent may be selected from benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and a combination thereof.

The lithium salt dissolved in the non-aqueous organic solvent supplies lithium ions in a battery, enables a basic operation of a rechargeable lithium battery, and improves transportation of the lithium ions between positive and negative electrodes. Examples of the lithium salt include at least one supporting salt selected from $LiPF_6$, $LiBF_4$, lithium difluoro(oxalate)borate (LiDFOB), $LiPO_2F_2$, $LiSbF_6$, $LiAsF_6$, $Li(FSO_2)_2N$ (lithium bis(fluorosulfonyl)imide (LiFSI), $LiC_4F_9SO_3$, $LiClO_4$, $LiAlO_2$, $LiAlCl_4$, $LiN(C_xF_{2x+1}SO_2)(C_yF_{2y+1}SO_2)$, wherein, x and y are each independently an integer selected from 1 to 20, LiCl, LiI, and $LiB(C_2O_4)_2$ (lithium bis(oxalato) borate: LiBOB).

The lithium salt may be used in a concentration in a range from about 0.1 M to about 2.0 M. If the lithium salt is included at the above concentration range, an electrolyte may have excellent performance and lithium ion mobility due to suitable or optimal electrolyte conductivity and viscosity.

$R^2$ in Chemical Formula 1 may be a substituted or unsubstituted C1 to C10 alkyl group.

$R^2$ in Chemical Formula 1 may be a substituted or unsubstituted C1 to C6 alkyl group.

$R^2$ in Chemical Formula 1 may be a substituted or unsubstituted C1 to C3 alkyl group.

The compound represented by Chemical Formula 1 may be represented by Chemical Formula 1-1.

Chemical Formula 1-1

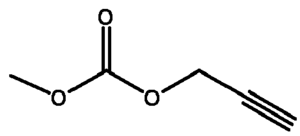

The compound represented by Chemical Formula 1 may be included in an amount of 0.05 to 5.0 parts by weight based on 100 parts by weight of the total electrolyte for a rechargeable lithium battery excluding additives (i.e. based on the total weight of lithium salt and non-aqueous organic solvent).

For example, the compound represented by Chemical Formula 1 may be included in an amount of 0.05 to 3.0 parts by weight based on 100 parts by weight of the total electrolyte for a rechargeable lithium battery excluding additives.

For example, the compound represented by Chemical Formula 1 may be included in an amount of 0.1 to 3.0 parts by weight, 0.3 to 3.0 parts by weight, 0.5 to 3.0 parts by weight, or 0.5 to 2.0 parts by weight based on 100 parts by weight of the total electrolyte for a rechargeable lithium battery excluding additives.

If the content range of the compound represented by Chemical Formula 1 is as described above, an increase in resistance at high temperatures may be prevented or reduced, and thus a rechargeable lithium battery having improved

cycle-life characteristics and output characteristics may be implemented.

The electrolyte may further include at least one other additive selected from vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propene sultone (PST), propane sultone (PS), lithium tetrafluoroborate ($LiBF_4$), lithium difluorophosphate ($LiPO_2F_2$), and 2-fluoro biphenyl (2-FBP).

By further including the aforementioned other additives, cycle-life may be further improved and/or gases generated from the positive electrode and the negative electrode may be suitably or effectively controlled during high-temperature storage.

The other additives may be included in an amount of about 0.2 to about 20 parts by weight, about 0.2 to about 15 parts by weight, or, for example about 0.2 to about 10 parts by weight based on the total weight of the electrolyte for a rechargeable lithium battery.

If the content of other additives is as described above, the increase in film resistance may be minimized or reduced, thereby contributing to the improvement of battery performance.

The positive electrode includes a positive electrode current collector and a positive electrode active material layer on the positive electrode current collector, and the positive electrode active material layer includes a positive electrode active material.

The positive electrode active material may include a cobalt-free lithium nickel manganese-based oxide.

For example, the lithium nickel manganese-based oxide may include at least one cobalt-free lithium composite oxide represented by Chemical Formula 4.

Chemical Formula 4 $Li_aNi_xMn_yM^1_zM^2_wO_{2\pm b}X_c$.

In Chemical Formula 4,

$0.5 \leq a < 1.8$, $0 \leq b \leq 0.1$, $0 \leq c \leq 0.1$, $0 \leq w < 0.1$, $0.6 \leq x < 1.0$, $0 < y < 0.4$, $0 < z < 0.1$, $w + x + y + z = 1$,
$M^1$ and $M^2$ are each independently one or more elements selected from Al, Mg, Ti, Zr, Sr, V, B, W, Mo, Si, Ba, Ca, Ce, Cr, Fe, and Nb, and X is one or more elements selected from S, F, P, and Cl.

As used herein, cobalt-free lithium composite oxide as a positive electrode active material means a positive electrode active material including nickel, manganese, etc. as a main component without including cobalt in the composition of the positive electrode active material.

The lithium composite oxide may have a coating layer on the surface, and/or may be mixed together with another compound having a coating layer. The coating layer may include at least one coating element compound selected from an oxide of the coating element, a hydroxide of the coating element, an oxyhydroxide of the coating element, an oxycarbonate of the coating element, and a hydroxy carbonate of the coating element. The compound for the coating layer may be amorphous or crystalline. The coating element included in the coating layer may include Mg, Al, K, Na, Ca, Si, Ti, V, Sn, Ge, Ga, B, As, Zr, or a mixture thereof. The coating process may include any suitable processes generally used in the art as long as it does not cause any side effects (e.g., substantially any undesirable side effect) on the properties of the positive electrode active material (e.g., spray coating, dipping), and therefore, a further detailed description thereof is not necessary here.

For example, the compound represented by Chemical Formula 4 may be represented by Chemical Formula 4-1.

Chemical Formula 4-1 $Li_aNi_{x1}Mn_{y1}Al_{z1}M^2_{w1}O_{2\pm b}X_c$

In Chemical Formula 4-1,

$$0.5 \leq a < 1.8,\ 0 \leq b \leq 0.1,\ 0 \leq c \leq 0.1,\ 0 \leq w1 < 0.1,\ 0.6 \leq x1 < 1.0,\ 0 < y1 < 0.4,\ 0 < z1 < 0.1,\ w1 + x1 + y1 + z1 = 1,$$

$M^2$ is one or more elements selected from Mg, Ti, Zr, Sr, V, B, W, Mo, Si, Ba, Ca, Ce, Cr, Fe, and Nb, and X is one or more elements selected from S, F, P, and Cl.

In some embodiments, in Chemical Formula 4-1, $0.6 \leq x1 \leq 0.9$, $0.1 \leq y1 < 0.4$, and $0 < z1 < 0.1$, or $0.6 \leq x1 \leq 0.8$, $0.2 \leq y1 < 0.4$, and $0 < z1 < 0.1$.

For example, in Chemical Formula 4-1, x1 may be $0.6 \leq x1 \leq 0.79$, y1 may be $0.2 \leq y1 \leq 0.39$, and z1 may be $0.01 \leq z1 < 0.1$.

A content of the positive electrode active material may be about 90 wt% to about 98 wt% based on the total weight of the positive electrode active material layer.

In some embodiments of the present disclosure, the positive electrode active material layer may include a binder. A content of the binder may be about 1 wt% to about 5 wt% based on the total weight of the positive electrode active material layer.

The binder improves binding properties of positive electrode active material particles with one another and with a positive electrode current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxidecontaining polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but are not limited thereto.

Al foil may be used as the positive electrode current collector, but is not limited thereto.

The negative electrode includes a negative electrode current collector and a negative electrode active material layer including a negative electrode active material formed on the negative electrode current collector.

The negative electrode active material may include a material that reversibly intercalates/deintercalates lithium ions, a lithium metal, a lithium metal alloy, a material capable of doping/dedoping lithium, and/or a transition metal oxide.

The material that reversibly intercalates/deintercalates lithium ions may be a carbon material which is any suitable, generally-used carbon-based negative electrode active material in a rechargeable lithium battery and examples thereof may be crystalline carbon, amorphous carbon, or a combination thereof. Examples of the crystalline carbon may be graphite such as amorphous, sheet-shape, flake, spherical shape or fiber-shaped natural graphite or artificial graphite. Examples of the amorphous carbon may be soft carbon or hard carbon, a mesophase pitch carbonized product, calcined coke, and the like.

The lithium metal alloy may be an alloy of lithium and a metal selected from Na, K, Rb, Cs, Fr, Be, Mg, Ca, Sr, Si, Sb, Pb, In, Zn, Ba, Ra, Ge, Al, and Sn.

The material capable of doping and dedoping lithium may be Si, a Si-C composite, $SiO_x$ ($0 < x < 2$), a Si-Q alloy (wherein Q is an element selected from an alkali metal, an alkaline-earth metal, a Group 13 element, a Group 14 element except for Si, a Group 15 element, a Group 16 element, a transition metal, a rare earth element, and a combination thereof), Sn, $SnO_2$, and/or Sn-$R^{11}$ alloy (wherein $R^{11}$ is an element selected from an alkali metal, an alkaline-earth metal, a Group 13 element, a Group 14 element except for Sn, a Group 15 element, a Group 16 element, a transition metal, a rare earth element, and a combination thereof, and not Sn), and at least one thereof may be mixed together with $SiO_2$.

The element Q and $R^{11}$ may be selected from Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn ($R^{11}$ does not comprise Sn), In, Tl, Ge, P, As, Sb, Bi, S, Se, Te, Po, and a combination thereof.

The transition metal oxide may include vanadium oxide, lithium vanadium oxide, and/or lithium titanium oxide.

In some embodiments, the negative electrode active material may include at least one selected from graphite and a Si composite.

The Si composite includes a core including Si-based particles and an amorphous carbon coating layer. For example, the Si-based particles may include at least one selected from silicon particles, a Si-C composite, $SiO_x$ ($0 < x \leq 2$), and an Si alloy.

For example, voids may be included in the central portion of the core including the Si-based particles, a radius of the central portion corresponds to 30% to 50% of a radius of the Si composite, an average particle diameter of the Si composite may be about 5 $\mu$m to about 20 $\mu$m, and an average particle diameter of the Si-based particles may be about 10 nm to about 200 nm.

In the present specification, the average particle diameter (D50) may be a particle size at 50% by volume in a cumulative size-distribution curve.

If the average particle diameter of the Si-based particles is within the above range, volume expansion occurring during charging and discharging may be suppressed or reduced, and a break in a conductive path due to particle crushing during charging and discharging may be prevented or reduced.

The core including the Si-based particles further includes amorphous carbon, and the central portion does not include amorphous carbon, and the amorphous carbon may exist only on the surface portion of the Si composite.

At this time, the surface portion means a region from the outermost surface of the central portion to the outermost surface of the Si composite.

In some embodiments, the Si-based particles may be substantially uniformly included throughout the Si composite, for example, may be present in a substantially uniform concentration in the central portion and surface portion.

The amorphous carbon may be soft carbon, hard carbon, a mesophase pitch carbonized product, calcined coke, or a combination thereof.

For example, the Si-C composite may include silicon particles and crystalline carbon.

The silicon particles may be included in an amount of about 1 to about 60 wt%, for example, about 3 to about

60 wt% based on the total weight of the Si-C composite.

The crystalline carbon may be, for example, graphite, and for example natural graphite, artificial graphite, or a combination thereof.

An average particle diameter of the crystalline carbon may be about 5 μm to about 30 μm.

If the negative electrode active material includes both graphite and Si composite, the graphite and Si composite may be included in a mixture form, and in some embodiments, the graphite and Si composite may be included in a weight ratio of about 99:1 to about 50:50.

For example, the graphite and Si composite may be included in a weight ratio of about 97:3 to about 80:20, or about 95:5 to about 80:20.

The amorphous carbon precursor may include a coal-based pitch, mesophase pitch, petroleum-based pitch, coal-based oil, petroleum-based heavy oil, and/or a polymer resin such as a phenol resin, a furan resin, and/or a polyimide resin.

In the negative electrode active material layer, the negative electrode active material may be included in an amount of about 95 wt% to about 99 wt% based on the total weight of the negative electrode active material layer.

In some embodiments of the present disclosure, the negative electrode active material layer further includes a binder, and optionally a conductive material (e.g., an electrically conductive material). In the negative electrode active material layer, a content of the binder may be about 1 wt% to about 5 wt% based on the total weight of the negative electrode active material layer. If the negative electrode active material layer includes a conductive material, the negative electrode active material layer includes about 90 wt% to about 98 wt% of the negative electrode active material, about 1 wt% to about 5 wt% of the binder, and about 1 wt% to about 5 wt% of the conductive material.

The binder improves binding properties of negative electrode active material particles with one another and with a negative electrode current collector. The binder includes a non-water-soluble binder, a water-soluble binder, or a combination thereof.

The non-water-soluble binder may be selected from polyvinylchloride, carboxylated polyvinylchloride, polyvinylfluoride, polyurethane, polytetrafluoroethylene, polyvinylidene fluoride, polyethylene, polypropylene, polyamideimide, polyimide, and a combination thereof.

The water-soluble binder may be a rubber-based binder and/or a polymer resin binder. The rubber-based binder may be selected from a styrene-butadiene rubber, an acrylated styrene-butadiene rubber (SBR), an acrylonitrile-butadiene rubber, an acrylic rubber, a butyl rubber, a fluorine rubber, and a combination thereof. The polymer resin binder may be selected from polytetrafluoroethylene, ethylenepropylene copolymer, polyethyleneoxide, polyvinylpyrrolidone, polyepichlorohydrin, polyphosphazene, polyacrylonitrile, polystyrene, an ethylenepropylenediene copolymer, polyvinylpyridine, chlorosulfonated polyethylene, latex, a polyester resin, an acrylic resin, a phenolic resin, an epoxy resin, polyvinyl alcohol, and a combination thereof.

If the water-soluble binder is used as binder in the negative electrode active material layer, a cellulose-based compound may be further used to provide viscosity as a thickener. The cellulose-based compound includes one or more of carboxymethyl cellulose, hydroxypropylmethyl cellulose, methyl cellulose, and alkali metal salts thereof. The alkali metals may be Na, K, and/or Li. Such a thickener may be included in an amount of about 0.1 parts by weight to about 3 parts by weight based on 100 parts by weight of the negative electrode active material.

The conductive material is included to provide electrode conductivity and any suitable electrically conductive material may be used as a conductive material unless it causes a chemical change (e.g., an undesirable chemical change). Examples of the conductive material include a carbon-based material such as natural graphite, artificial graphite, carbon black, acetylene black, ketjen black, a carbon fiber, and the like; a metal-based material of a metal powder and/or a metal fiber including copper, nickel, aluminum silver, and the like; a conductive polymer such as a polyphenylene derivative; or a mixture thereof.

The negative electrode current collector may be selected from a copper foil, a nickel foil, a stainless steel foil, a titanium foil, a nickel foam, a copper foam, a polymer substrate coated with a conductive metal (e.g., an electrically conductive metal), and a combination thereof.

A separator may exist between the positive electrode and the negative electrode depending on the type (or kind) of the rechargeable lithium battery. Such a separator may include polyethylene, polypropylene, polyvinylidene fluoride, or multi-layers thereof such as a polyethylene/polypropylene double-layered separator, a polyethylene/polypropylene/polyethylene triple-layered separator, and a polypropylene/polyethylene/polypropylene triple-layered separator.

The rechargeable lithium battery may have a charging upper limit voltage of greater than or equal to about 4.35 V. For example, the charging upper limit voltage may be about 4.35 V to about 4.55 V.

Hereinafter, examples of the present disclosure and comparative examples are described. These examples, however, are not in any sense to be interpreted as limiting the scope of the present disclosure.

**Manufacture of Rechargeable Lithium Battery Cell**

**Example 1**

$LiNi_{0.75}Mn_{0.23}Al_{0.02}O_2$ as a positive electrode active material, polyvinylidene fluoride as a binder, and acetylene black as a conductive material were mixed together in a weight ratio of 96:3:1, respectively, and were dispersed in N-methyl pyrrolidone to prepare a positive electrode active material slurry.

The positive electrode active material slurry was coated on a 15 μm-thick Al foil, dried at 100 °C, and then pressed to prepare a positive electrode.

As a negative electrode active material, a mixture of artificial graphite and a Si composite in a weight ratio of 93:7 was used, and the negative electrode active material, a styrene-butadiene rubber binder, and carboxymethyl cellulose were mixed together in a weight ratio of 98:1:1, respectively, and dispersed in distilled water to prepare a negative electrode active material slurry.

The Si composite included a core including artificial graphite and silicon particles, and a coal-based pitch is coated on the surface of the core.

The negative electrode active material slurry was coated on a 10 μm-thick Cu foil, dried at 100 °C, and then pressed to prepare a negative electrode.

An electrode assembly was prepared by assembling the prepared positive and negative electrodes and a polyethylene separator having a thickness of 25 μm, and an electrolyte was injected to prepare a rechargeable lithium battery cell.

A composition of the electrolyte is as follows.

Composition of Electrolyte

Lithium salt: 1.5 M $LiPF_6$
Non-aqueous organic solvent: ethylmethyl carbonate : dimethyl carbonate (EMC:DMC= volume ratio of 20:80)
Additives: 1 part by weight of the compound represented by Chemical Formula 1-1, 10 parts by weight of fluoroethylene carbonate (FEC) / 0.5 parts by weight of succinonitrile (SN)

Chemical Formula 1-1

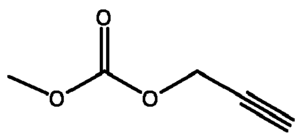

In the composition of the electrolyte, "parts by weight" means a relative weight of additives based on 100 parts by weight of the total electrolyte (lithium salt+non-aqueous organic solvent) excluding additives.

**Comparative Example 1**

A rechargeable lithium battery cell was manufactured in substantially the same manner as in Example 1, except that the compound represented by Chemical Formula 1-1 was not added to the composition of the electrolyte.

**Comparative Example 2**

A rechargeable lithium battery cell was manufactured in substantially the same manner as in Example 1, except that 20 wt% of ethylene carbonate based on the total weight of the non-aqueous organic solvent in the composition of the electrolyte was added.

**Examples 2 to 4**

Each of rechargeable lithium battery cells was manufactured in substantially the same manner as in Example 1, except that the content of the compound represented by Chemical Formula 1-1 was respectively changed into 0.5 parts by weight, 1.5 parts by weight, and 2 parts by weight.

**Comparative Example 3**

A rechargeable lithium battery cell was manufactured in substantially the same manner as in Example 1, except that 1 part by weight of a compound represented by Chemical Formula a in the composition of the electrolyte was added instead of the compound represented by Chemical Formula 1-1.

Chemical Formula a

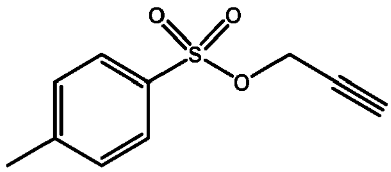

**Example 5**

A rechargeable lithium battery cell was manufactured in substantially the same manner as in Example 1, except that the non-aqueous organic solvent was changed into 100 volume% of dimethyl carbonate, i.e., only dimethyl carbonate was used as the non-aqueous organic solvent.

**Comparative Examples 4 to 6**

Each of rechargeable lithium battery cells was manufactured in substantially the same manner as in Example 1 and Comparative Examples 1 and 2, respectively except that the positive electrode active material was changed into $LiCoO_2$.

**Comparative Examples 7 to 9**

Each of rechargeable lithium battery cells was manufactured in substantially the same manner as in Example 1 and Comparative Examples 1 and 2, respectively except that the positive electrode active material was changed into $LiNi_{0.5}Co_{0.2}Al_{0.3}O_2$.

**Comparative Example 10 to 12**

Each of rechargeable lithium battery cells was manufactured in substantially the same manner as in Example 1 and Comparative Examples 1 and 2, respectively except that the positive electrode active material was changed into $LiNi_{0.8}Co_{0.1}Mn_{0.1}O_2$.

**Examples 6 and 7 and Comparative Example 13**

Each of rechargeable lithium battery cells was manufactured in substantially the same manner as in Example 1, except that the volume ratio of mixing ethylmethyl carbonate and dimethyl carbonate was changed respectively into 30: 70 (Example 6), 40 : 60 (Example 7), and 70 : 30 (Comparative Example 13).

Each composition is shown in Table 1.

Table 1

| | Positive electrode active material | Composition of the electrolyte |
|---|---|---|
| Ex. 1 | $LiNi_{0.75}Mn_{0.23}Al_{0.02}O_2$ | 1.5 M $LiPF_6$ in EMC/DMC 2/8<br>Chemical Formula 1-1 1 part by weight<br>FEC 10 parts by weight<br>SN 0.5 parts by weight |

(continued)

| | Positive electrode active material | Composition of the electrolyte |
|---|---|---|
| Ex. 2 | | 1.5 M $LiPF_6$ in EMC/DMC 2/8<br>Chemical Formula 1-1 0.5 parts by weight<br>FEC 10 parts by weight<br>SN 0.5 parts by weight |
| Ex. 3 | | 1.5 M $LiPF_6$ in EMC/DMC 2/8<br>Chemical Formula 1-1 1.5 parts by weight<br>FEC 10 parts by weight<br>SN 0.5 parts by weight |
| Ex. 4 | | 1.5 M $LiPF_6$ in EMC/DMC 2/8<br>Chemical Formula 1-1 2 parts by weight<br>FEC 10 parts by weight<br>SN 0.5 parts by weight |
| Ex. 5 | | 1.5 M $LiPF_6$ in EMC/DMC 0/100<br>Chemical Formula 1-1 1 part by weight<br>FEC 10 parts by weight<br>SN 0.5 parts by weight |
| Ex. 6 | | 1.5 M $LiPF_6$ in EMC/DMC 3/7<br>Chemical Formula 1-1 1 part by weight<br>FEC 10 parts by weight<br>SN 0.5 parts by weight |
| Ex. 7 | | 1.5 M $LiPF_6$ in EMC/DMC 4/6<br>FEC 10 parts by weight<br>SN 0.5 parts by weight<br>Chemical Formula 1-1 1 part by weight |
| Comp. Ex. 1 | $LiNi_{0.75}Mn_{0.23}Al_{0.02}O_2$ | 1.5 M $LiPF_6$ in EMC/DMC 2/8<br>FEC 10 parts by weight<br>SN 0.5 parts by weight |
| Comp. Ex. 2 | | 1.5 M $LiPF_6$ in EMC/DMC 2/8<br>FEC 10 parts by weight<br>SN 0.5 parts by weight<br>EC 20 wt% |
| Comp. Ex. 3 | | 1.5 M $LiPF_6$ in EMC/DMC 2/8<br>Chemical Formula a 1 part by weight<br>FEC 10 parts by weight<br>SN 0.5 parts by weight |

(continued)

| | Positive electrode active material | Composition of the electrolyte |
|---|---|---|
| Comp. Ex. 4 | $LiCoO_2$ | 1.5 M $LiPF_6$ in EMC/DMC 2/8<br>FEC 10 parts by weight<br>SN 0.5 parts by weight |
| Comp. Ex. 5 | | 1.5 M $LiPF_6$ in EMC/DMC 2/8<br>FEC 10 parts by weight<br>SN 0.5 parts by weight<br>EC 20 wt% |
| Comp. Ex. 6 | | 1.5 M $LiPF_6$ in EMC/DMC 2/8<br>Chemical Formula 1-1 1 part by weight<br>FEC 10 parts by weight<br>SN 0.5 parts by weight |
| Comp. Ex. 7 | $LiNi_{0.5}Co_{0.2}Al_{0.3}O_2$ | 1.5 M $LiPF_6$ in EMC/DMC 2/8<br>FEC 10 parts by weight<br>SN 0.5 parts by weight |
| Comp. Ex. 8 | | 1.5 M $LiPF_6$ in EMC/DMC 2/8<br>FEC 10 parts by weight<br>SN 0.5 parts by weight<br>EC 20 wt% |
| Comp. Ex. 9 | | 1.5 M $LiPF_6$ in EMC/DMC 2/8 |
| | | Chemical Formula 1-1 1 part by weight<br>FEC 10 parts by weight<br>SN 0.5 parts by weight |
| Comp. Ex. 10 | $LiNi_{0.8}Co_{0.1}Mn_{0.1}O_2$ | 1.5 M $LiPF_6$ in EMC/DMC 2/8<br>FEC 10 parts by weight<br>SN 0.5 parts by weight |
| Comp. Ex. 11 | | 1.5 M $LiPF_6$ in EMC/DMC 2/8<br>FEC 10 parts by weight<br>SN 0.5 parts by weight<br>EC 20 wt% |
| Comp. Ex. 12 | | 1.5 M $LiPF_6$ in EMC/DMC 2/8<br>Chemical Formula 1-1 1 part by weight<br>FEC 10 parts by weight<br>SN 0.5 parts by weight |
| Comp. Ex. 13 | $LiNi_{0.75}Mn_{0.23}Al_{0.02}O_2$ | 1.5 M $LiPF_6$ in EMC/DMC 7/3<br>Chemical Formula 1-1 1 parts by weight<br>FEC 10 parts by weight<br>SN 0.5 parts by weight |

**Evaluation 1: Evaluation of Storage Characteristics at High Temperature**

The rechargeable lithium battery cells according to Examples 1 to 7 and Comparative Examples 1 to 13 were measured with respect to initial DC internal resistance (DCIR) as $\triangle V/\triangle I$ (change in voltage/change in current), and after setting a maximum energy state inside the rechargeable lithium battery cells to a fully charged state (SOC 100%), the rechargeable lithium battery cells were stored at a high temperature (60 °C) for 30 days and then, measured again with respect to DC internal resistance to calculate a DCIR increase rate (%) according to Equation 1, and the results are

shown in Tables 2, 3, and 6.

Equation 1

$$\text{DCIR increase rate} = \{(\text{DCIR after 30 days}) / (\text{initial DCIR})\} \times 100$$

**Evaluation 2: Evaluation of Cycle-life Characteristics at High Temperature**

The rechargeable lithium battery cells according to Example 1 and Comparative Examples 1 to 3 were once charged and discharged at 0.2 C and then, measured with respect to charge and discharge capacity.

The rechargeable lithium battery cells according to Example 1 and Comparative Examples 1 to 3 were charged to an upper limit voltage of 4.35 V and discharged at 0.2 C to 2.5 V under a constant current condition to measure initial discharge capacity.

Then, the rechargeable lithium battery cells were 200 cycles charged at 0.33 C (CC/CV, 4.35 V, 0.025 C cut-off) and discharged at 1.0 C (CC, 2.5 V cut-off) at 45 °C and then, measured with respect to discharge capacity. A ratio of the discharge capacity to the initial discharge capacity was calculated and provided as a capacity recovery rate (%, recovery) in Table 5, and an increase rate relative to Comparative Example 2 was calculated according to Equation 2. The results are shown in Table 5.

Equation 2

$$\text{Increase rate relative to Comparative Example 2} = \{(\text{capacity recovery rate})/(\text{capacity recovery rate of Comparative Example 2})\} \times 100$$

**Evaluation 3**: **Measurement of Gas Generation after High-temperature Storage**

The rechargeable lithium battery cells according to Examples 1 and 5 and Comparative Examples 1 to 3 were left at 60 °C for 7 days and then, measured with respect to an amount of generated gas (mL) at the $1^{st}$ day and the $7^{th}$ day through a refinery gasanalysis (RGA) to calculate an increase rate according to Equation 3, and the results are shown in Table 4.

Equation 3

$$\text{Increase rate} = \{(\text{gas generation after 7 days})/(\text{gas generation on first day})\} \times 100$$

Table 2

| | Initial DCIR (mOhm) | DCIR (mOhm) after storage at high temperature (60 °C) for 30 days | DCIR Increase rate (60 °C, 30 days) (%) |
|---|---|---|---|
| Ex. 1 | 42.14 | 45.42 | 108 |
| Ex. 2 | 42.28 | 44.07 | 104 |
| Ex. 3 | 42.44 | 44.24 | 104 |
| Ex. 4 | 42.99 | 45.04 | 105 |
| Ex. 5 | 41.82 | 44.55 | 107 |
| Comp. Ex. 1 | 42.41 | 46.87 | 111 |
| Comp. Ex. 2 | 42.31 | 53.40 | 126 |

(continued)

| | Initial DCIR (mOhm) | DCIR (mOhm) after storage at high temperature (60 °C) for 30 days | DCIR Increase rate (60 °C, 30 days) (%) |
|---|---|---|---|
| Comp. Ex. 3 | 43.98 | 55.74 | 127 |

Table 3

| Positive electrode active material | | Initial DCIR (mOhm) | DCIR (mOhm) after storage at high temperature (60 °C) for 30 days | DCIR increase rate (60 °C, 30 days) (%) |
|---|---|---|---|---|
| $LiNi_{0.75}Mn_{0.23}Al_{0.02}O_2$ | Ex. 1 | 42.14 | 45.42 | 108 |
| | Comp. Ex. 1 | 42.41 | 46.87 | 111 |
| | Comp. Ex. 2 | 42.31 | 53.40 | 126 |
| $LiCoO_2$ | Comp. Ex. 4 | 43.50 | 53.31 | 123 |
| | Comp. Ex. 5 | 43.30 | 52.98 | 122 |
| | Comp. Ex. 6 | 43.15 | 53.10 | 123 |
| $LiNi_{0.5}Co_{0.2}Al_{0.3}O_2$ | Comp. Ex. 7 | 43.26 | 52.10 | 120 |
| | Comp. Ex. 8 | 43.37 | 52.62 | 121 |
| | Comp. Ex. 9 | 43.43 | 51.91 | 120 |
| $LiNi_{0.8}Co_{0.1}Mn_{0.1}O_2$ | Comp. Ex. 10 | 43.68 | 49.88 | 114 |
| | Comp. Ex. 11 | 44.08 | 50.02 | 113 |
| | Comp. Ex. 12 | 44.45 | 50.09 | 113 |

Table 4

| | Amount of gas generated after storage at high temperature (60 °C) | | |
|---|---|---|---|
| | $1^{st}$ day (mL) | $7^{th}$ day (mL) | Increase rate (%) |
| Ex. 1 | 1.10 | 1.57 | 143 |
| Ex. 5 | 1.07 | 1.48 | 138 |
| Comp. Ex. 1 | 1.22 | 1.81 | 148 |
| Comp. Ex. 2 | 1.40 | 3.80 | 271 |
| Comp. Ex. 3 | 1.42 | 3.90 | 275 |

Table 5

| | Capacity recovery rate (45 °C, 200th cy, %) | Increase rate relative to Comparative Example 2 (%) |
|---|---|---|
| Ex. 1 | 95.3 | 102 |
| Ex. 5 | 96.0 | 102 |
| Comp. Ex. 1 | 94.5 | 101 |
| Comp. Ex. 2 | 93.8 | 100 |
| Comp. Ex. 3 | 94.2 | 100 |

Table 6

| | Initial DCIR (mOhm) | DCIR (mOhm) after storage at high temperature (60 °C) for 30 days | DCIR increase rate (60 °C, 30 days) (%) |
|---|---|---|---|
| Ex. 1 | 42.14 | 45.42 | 108 |
| Ex. 5 (EMC: DMC=0:100) | 41.82 | 44.55 | 107 |
| Ex. 6 (EMC: DMC=30:70) | 41.24 | 44.98 | 109 |
| Ex. 7 (EMC: DMC=40:60) | 41.88 | 45.37 | 108 |
| Comp. Ex. 2 EC 20 wt% | 42.31 | 53.40 | 126 |
| Comp. Ex. 13 (EMC: DMC=70: 30) | 43.33 | 54.45 | 126 |

Referring to Tables 2 to 6, DCIR increase rates were decreased by the combinations of an electrolyte and a cobalt-free positive electrode active material according to embodiments of the present disclosure, thereby improving high-temperature storage characteristics and high-temperature charge and discharge characteristics.
The rechargeable lithium battery cells according to the examples exhibited a significantly reduced amount of gas generated after stored at a high temperature.

**Description of Symbols**

100: rechargeable lithium battery
112: negative electrode
113: separator
114: positive electrode
120: battery case
140: sealing member

**Claims**

**1.** A rechargeable lithium battery (100), comprising:

an electrolyte comprising a non-aqueous organic solvent, a lithium salt, and an additive;
a positive electrode (114) comprising a positive electrode active material; and
a negative electrode (112) comprising a negative electrode active material,

wherein the non-aqueous organic solvent comprises less than 5 wt% of ethylene carbonate based on the total weight of the non-aqueous organic solvent,
the positive electrode active material comprises a lithium nickel manganese-based oxide, and
the additive comprises a compound represented by Chemical Formula 1:

Chemical Formula 1

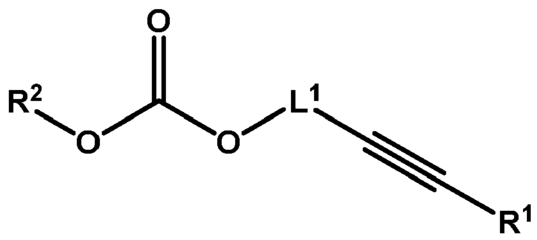

wherein, in Chemical Formula 1,
$L^1$ is a substituted or unsubstituted C1 to C3 alkylene group,
$R^1$ is hydrogen or a substituted or unsubstituted C1 to C10 alkyl group, and
$R^2$ is a substituted or unsubstituted C1 to C10 alkyl group, a substituted or unsubstituted C2 to C10 alkenyl group, a substituted or unsubstituted C2 to C10 alkynyl group, or a substituted or unsubstituted C6 to C20 aryl group,
wherein "substituted" refers to replacement of at least one hydrogen of a substituent by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

**2.** The rechargeable lithium battery as claimed in claim 1, wherein
the non-aqueous organic solvent is composed of only a chain carbonate.

**3.** The rechargeable lithium battery as claimed in claim 2, wherein

the chain carbonate is represented by Chemical Formula 2:

Chemical Formula 2

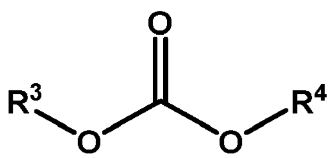

wherein, in Chemical Formula 2
$R^3$ and $R^4$ are each independently a substituted or unsubstituted C1 to C20 alkyl group, wherein "substituted" refers to replacement of at least one hydrogen of a substituent by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

**4.** The rechargeable lithium battery as claimed in claim 3, wherein
the non-aqueous organic solvent comprises mixed solvent of at least two solvent selected from dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

**5.** The rechargeable lithium battery as claimed in claim 3 or 4, wherein
the non-aqueous organic solvent is dimethyl carbonate (DMC) or a combination of ethylmethyl carbonate (EMC)

and dimethyl carbonate (DMC).

6. The rechargeable lithium battery of any one of the preceding claims, wherein $R^2$ in Chemical Formula 1 is a substituted or unsubstituted C1 to C10 alkyl group.

7. The rechargeable lithium battery as claimed in claim 6, wherein $R^2$ in Chemical Formula 1 is a substituted or unsubstituted C1 to C6 alkyl group.

8. The rechargeable lithium battery as claimed in claim 7, wherein $R^2$ in Chemical Formula 1 is a substituted or unsubstituted C1 to C3 alkyl group.

9. The rechargeable lithium battery as claimed in claim 1, wherein the compound represented by Chemical Formula 1 is represented by Chemical Formula 1-1:

Chemical Formula 1-1

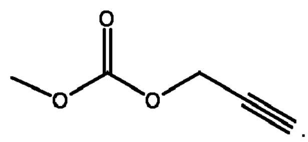

10. The rechargeable lithium battery of any one of the preceding claims, wherein the compound represented by Chemical Formula 1 is included in an amount of 0.05 to 5.0 parts by weight based on 100 parts by weight of the total electrolyte for a rechargeable lithium battery excluding additives.

11. The rechargeable lithium battery of any one of the preceding claims, wherein the electrolyte further comprises at least one other additive selected from vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propene sultone (PST), propane sultone (PS), lithium tetrafluoroborate ($LiBF_4$), lithium difluorophosphate ($LiPO_2F_2$), and 2-fluoro biphenyl (2-FBP).

12. The rechargeable lithium battery of any one of the preceding claims, wherein

the lithium nickel manganese-based oxide comprises a cobalt-free lithium composite oxide represented by Chemical Formula 4:

Chemical Formula 4 $Li_aNi_xMn_yM^1_zM^2_wO_{2\pm b}X_c$

wherein, in Chemical Formula 4,
$0.5 \leq a < 1.8$, $0 \leq b \leq 0.1$, $0 \leq c \leq 0.1$, $0 \leq w < 0.1$, $0.6 \leq x < 1.0$, $0 < y < 0.4$,$0 < z < 0.1$, $w + x + y + z = 1$,
$M^1$ and $M^2$ are each independently one or more elements selected from Al, Mg, Ti, Zr, Sr, V, B, W, Mo, Si, Ba, Ca, Ce, Cr, Fe, and Nb, and X is one or more elements selected from S, F, P, and Cl.

13. The rechargeable lithium battery as claimed in claim 12, wherein Chemical Formula 4 is represented by Chemical Formula 4-1:

Chemical Formula 4-1 $Li_aNi_{x1}Mn_{y1}Al_{z1}M^2_{w1}O_{2\pm b}X_c$

wherein, in Chemical Formula 4-1,

$0.5 \leq a < 1.8$, $0 \leq b \leq 0.1$, $0 \leq c \leq 0.1$, $0 \leq w1 < 0.1$, $0.6 \leq x1 < 1.0$, $0 < y1 < 0.4$, $0 < z1 < 0.1$, $w1 + x1 + y1 + z1 = 1$,
$M^2$ is one or more elements selected from Mg, Ti, Zr, Sr, V, B, W, Mo, Si, Ba, Ca, Ce, Cr, Fe, and Nb, and X is one or more elements selected from S, F, P, and Cl.

14. The rechargeable lithium battery as claimed in claim 13, wherein in Chemical Formula 4-1, x1 is $0.6 \leq x1 \leq 0.79$, y1 is $0.2 \leq y1 \leq 0.39$, and z1 is $0.01 \leq z1 < 0.1$.

**15.** The rechargeable lithium battery of any one of the preceding claims, wherein the negative electrode active material comprises at least one selected from graphite and a Si composite.

FIG. 1

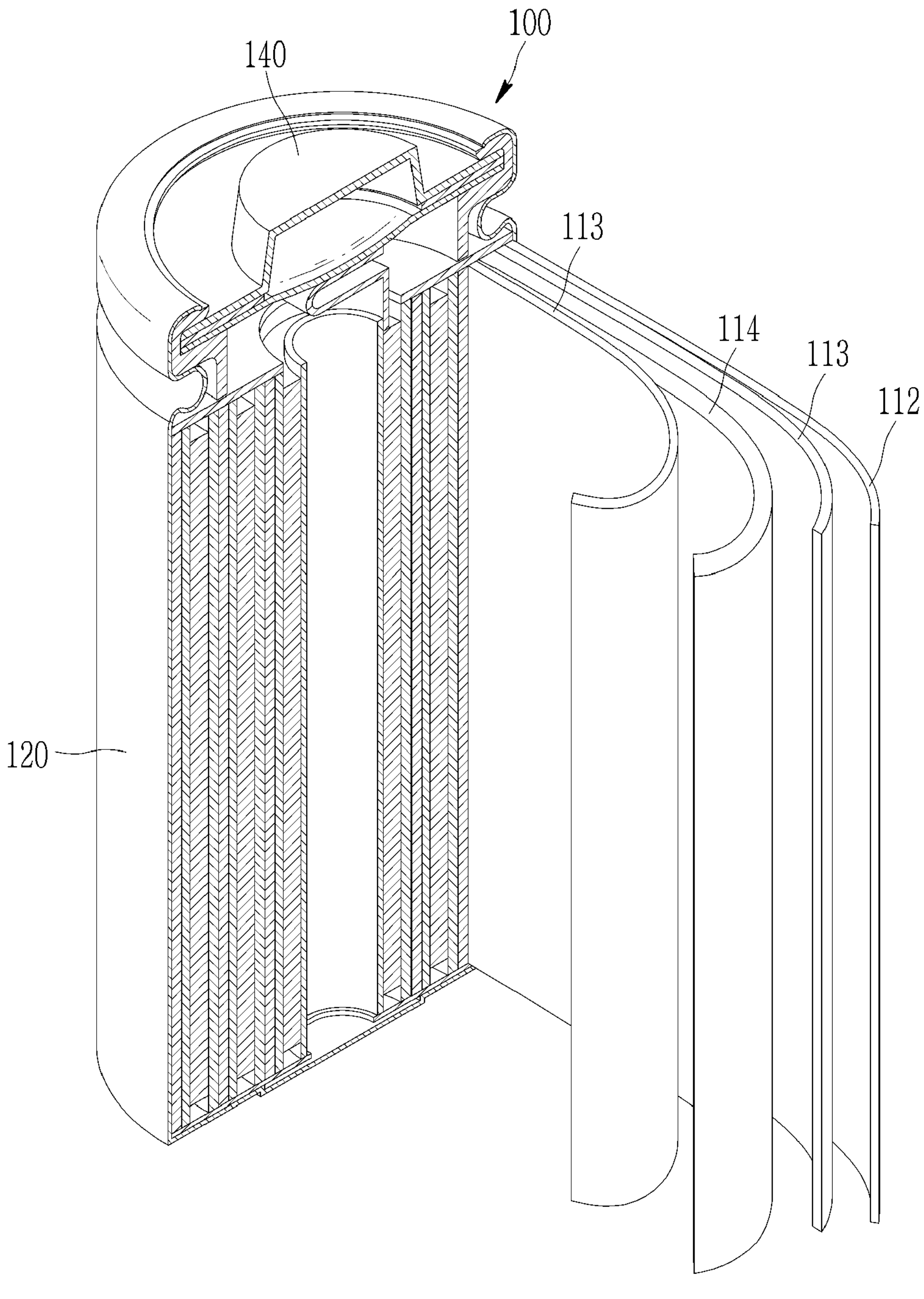

Europäisches Patentamt
European Patent Office
Office européen des brevets

# EUROPEAN SEARCH REPORT

Application Number
EP 23 20 9084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2020/163879 A1 (SOUTH 8 TECHNOLOGIES INC [GB]) 13 August 2020 (2020-08-13) * Claims 12, 19 * ----- | 1-15 | INV. H01M4/485 H01M10/0525 H01M10/0567 H01M10/0569 |
| A | US 6 927 001 B1 (HAMAMOTO TOSHIKAZU [JP] ET AL) 9 August 2005 (2005-08-09) * claim 1 * ----- | 1-15 | |
| | | | TECHNICAL FIELDS SEARCHED (IPC) |
| | | | H01M |

The present search report has been drawn up for all claims

1

| Place of search | Date of completion of the search | Examiner |
|---|---|---|
| Munich | 22 May 2024 | Bettio, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 20 9084

This annex lists the patent family members relating to the patent documents cited in the above-mentioned European search report. The members are as contained in the European Patent Office EDP file on
The European Patent Office is in no way liable for these particulars which are merely given for the purpose of information.

22-05-2024

| Patent document cited in search report | Publication date | Patent family member(s) | Publication date |
|---|---|---|---|
| WO 2020163879 A1 | 13-08-2020 | US 2021313611 A1 | 07-10-2021 |
| | | WO 2020163879 A1 | 13-08-2020 |
| US 6927001 B1 | 09-08-2005 | CN 1277468 A | 20-12-2000 |
| | | JP 3823683 B2 | 20-09-2006 |
| | | JP 2001043895 A | 16-02-2001 |
| | | US 6927001 B1 | 09-08-2005 |

EPO FORM P0459

For more details about this annex : see Official Journal of the European Patent Office, No. 12/82